# EUROPEAN PATENT APPLICATION

(11) **EP 3 434 273 A1**
(43) Date of publication of application: **30.01.2019**
(21) Application number: 18175808.7
(22) Date of filing: 04.06.2018
(51) Int. Cl.: A61K 31/546, A61P 25/28

(54) **CEFTRIAXONE FOR USE IN THE TREATMENT OF DEMENTIA WITH LEWY BODIES**

(30) Priority: 27.07.2017 TW 106125321
(71) Applicant: BrainX Corporation, Savalalo, Apia (WS)
(72) Inventor: HO, Ying-Jui, Taichung City (TW)
(74) Representative: Lang, Christian

(57) **Abstract**

Ceftriaxone is to be administrated to a subject suffered from dementia with Lewy bodies (DLB) to treat the DLB syndromes such as cognitive decline and neurological deficit in the subject thereof. Ceftriaxone can be parenterally or orally administrated to the subject in a dosage of 1.5-35 mg/kg/day.

## Description

### CROSS REFERENCE TO RELATED APPLICATIONS

The application claims the benefit of Taiwan application serial No. 106125321, filed on July 27, 2017, and the entire contents of which are incorporated herein by reference.

### BACKGROUND OF THE INVENTION

### 1. Field of the Invention

The present invention generally relates to ceftriaxone and, more particularly, to ceftriaxone for use in the treatment of dementia with Lewy bodies.

### 2. Description of the Related Art

Dementia with Lewy bodies (DLB) is a common subtype of neurodegenerative dementia. Patients with DLB have symptoms such as gradual cognitive dysfunction, motor symptom and visual hallucination, which may severely affect the quality of life, social activities and working performance.

Clinically, there is only medicament for relieving partial symptoms in the DLB patients. Yet, no medicament for effectively treating DLB is developed. Thus, it is necessary to develop ceftriaxone for use in the treatment of DLB.

### SUMMARY OF THE INVENTION

It is therefore the objective of the present invention to provide ceftriaxone for use in the treatment of dementia with Lewy bodies using ceftriaxone.

One embodiment of the invention discloses ceftriaxone for use in the treatment of dementia with Lewy bodies (DLB). Ceftriaxone is to be administrated to a subject in need thereof to treat the syndromes such as cognitive decline and neurological deficit in the subject in need thereof. Accordingly, by the administration of ceftriaxone, the neurogenesis is improved in the hippocampal dentate gyrus (DG) and substantia nigra pars reticulata (SNr), and the DLB syndromes such as cognitive decline (on recognition ability and learning ability) and neurological deficit are therefore improved.

In a preferred form shown, ceftriaxone is to be administrated to the subject in a dosage of 1.5-35 mg/kg/day. As an example, ceftriaxone is to be administrated to the subject in a dosage of 1.62-32.4 mg/kg/day. Preferably, ceftriaxone is to be administrated to the subject in a dosage of 1.62-16.2 mg/kg/day. In particular, ceftriaxone is to be administrated to the subject in a dosage of 1.62-8.1 mg/kg/day.

In a preferred form shown, ceftriaxone is to be parenterally or orally administrated to the subject. For example, ceftriaxone is to be administrated to the subject by intravenous injection, intramuscular injection, intraperitoneal injection, transdermal administration, sublingual administration or hebulization administration.

### BRIEF DESCRIPTION OF THE DRAWINGS

The present invention will become more fully understood from the detailed description given hereinafter and the accompanying drawings which are given by way of illustration only, and thus are not limitative of the present invention, and wherein:
FIG. 1 depicts a diagram illustrating the chemical structure of ceftriaxone.
FIG. 2a depicts a schematic diagram illustrating an open box used for the exposure session in trial (B).
FIG. 2b depicts a schematic diagram illustrating the open box used for a test session in trial (B).
FIG. 3 depicts a bar chart illustrating the percentage of time exploring object in trial (B).
FIG. 4a depicts a schematic diagram illustrating the conditioned stimulus (CS) presentation when a rat "M" under the test, is placed in a compartment C1 of a shuttle box in trial (C).
FIG. 4b depicts a schematic diagram illustrating the unconditioned stimulus (UCS) is given to the rat "M" under the test, if the rat "M" under the test does not move to the opposite compartment C2 of the shuttle box during the CS presentation in trial (C).
FIG. 4c depicts a schematic diagram illustrating the rat "M" under the test avoids the UCS by moving to the compartment C2 of the shuttle box during the CS presentation in trial (C).
FIG. 5 depicts a bar chart illustrating the percentage of active avoidance in trial (C).
FIG. 6 depicts a bar chart illustrating the percentage of pyramidal neuron (Nissl-stained cells) in a predetermined area in the hippocampal CA1 area in trial (D).
FIG. 7a depicts a bar chart illustrating the area of pyramidal neuron (Nissl-stained cells) in the predetermined area in the hippocampal dentate gyrus (DG) in trial (D).
FIG. 7b depicts a bar chart illustrating the density of α-synuclein-positive cells in the predetermined area in the DG in trial (D).
FIG. 7c depicts a bar chart illustrating the number of BrdU-positive cells in the predetermined area in DG in trial (D).
FIG. 8 depicts a bar chart illustrating the optical density of tyrosine hydroxylase-immunoreactive fibers in the predetermined area in striatum in trial (D).
FIG. 9 depicts a bar chart illustrating the density of tyrosine hydroxylase-immunoreactive cells (dopaminergic neurons) in the predetermined area in the substantia nigra pars compacta (SNc) in trial (D).
FIG. 10 depicts a bar chart illustrating the number of BrdU-positive cells in the predetermined area in the substantia nigra pars reticulata (SNr) in trial (D).

### DETAILED DESCRIPTION OF THE INVENTION

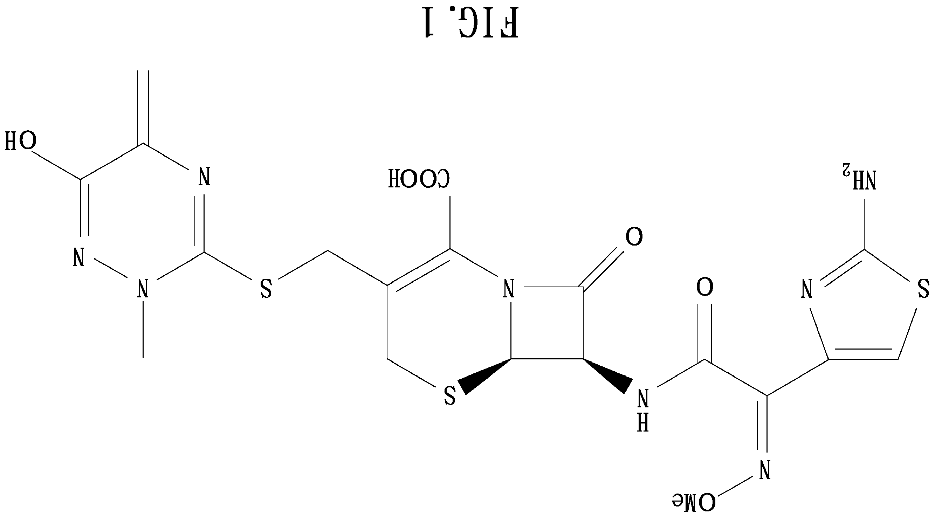

Ceftriaxone with a chemical structure shown in FIG. 1 is an antibiotic belonging to the third-generation cephalosporin and has broad-spectrum activity against Gram-positive bacteria and Gram-negative bacteria.

In the present invention, ceftriaxone can be administrated to the subject in need thereof, treating the symptoms such as cognitive decline and neurological deficit in the subject in need thereof. Therefore, ceftriaxone can be used in combination with pharmaceutical acceptable vehicles, excipients, salts or other nutrients, forming a pharmaceutical composition. In addition, ceftriaxone can be further manufactured into any oral type that is easy to take, such as pastil, capsule, powder, pill or solution.

Moreover, ceftriaxone can be administrated to the subject in need thereof via any suitable routes. For example, ceftriaxone can be parenterally or orally administrated to the subject, such as by intravenous injection (IV injection), intramuscular injection (IM injection), intraperitoneal injection (IP injection), transdermal administration, sublingual administration or hebulization administration.

In this embodiment, ceftriaxone is administrated to the subject in need thereof in a dosage of 1.5-35 mg/kg/day. As an example, ceftriaxone is administrated to the subject in a dosage of 1.62-32.4 mg/kg/day. Preferably, ceftriaxone is administrated to the subject in a dosage of 1.62-16.2 mg/kg/day. In particular, ceftriaxone is administrated to the subject in a dosage of 1.62-8.1 mg/kg/day. However, the dosage of ceftriaxone may vary according to the differences of the subject in need thereof, the sequence of administration and the routes of administration, which can be appreciated by a person having ordinary skill in the art.

In order to evaluate whether the administration of ceftriaxone can effectively treat the DLB syndromes such as cognitive decline and neurological deficit in the subject in need thereof, the following trials are carried out.

### Trial (A). Induction of DLB Rats

Wistar male rats (12 week-old, weight ∼420±30 grams) purchased from BioLASCO Taiwan Co., Ltd are used. The rats are housed in an animal room with constant temperature of 21-25°C, where is kept on a 12-hours light and 12-hours dark cycle. The rats are housed and kept on free diet and water.

On the 0^{th} day, the rats are anesthetized, followed by bilaterally infusing with β-amyloid protein in the prefrontal cortex and unilaterally infusing with α-synuclein (lentiviral vectors cloning with SNCA genes) in the ventricle to model the DLB rats.

Starting on the 1^{st} day, referring to TABLE 1, saline (group A2, 1 mL/kg/day, IP injection) and ceftriaxone (group A3, 100 mg/kg/day, IP injection) are administrated to the DLB rats in the corresponding groups. Saline is also used to be administrated to the normal rats of group A1. Moreover, saline and ceftriaxone are administrated for 27 days (from the 1^{st} day to the 27^{th} day).

**TABLE 1**

| Groups | Rats | Treatment |
|---|---|---|
| A1 | Normal rats | Saline |
| A2 | DLB rats | Saline |
| A3 | DLB rats | Ceftriaxone |

### Trial (B). Object Recognition Test

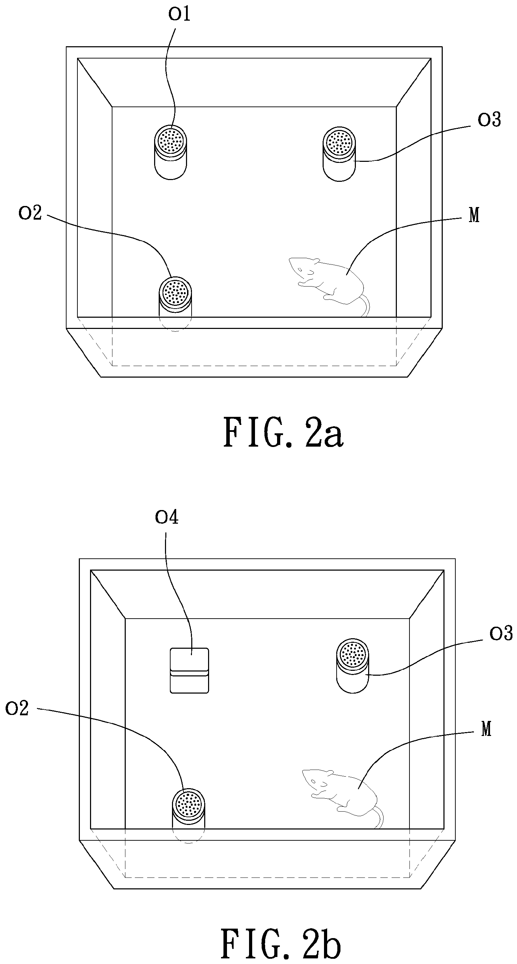

Recognition ability of the rats of groups A1-A3 is measured using the object recognition test (Ho et al., Behav Brain Res 268: 177-184, 2014). Specifically, on the 25^{th} & 26^{th} day, the rat "M" under the test is placed in an open box, shown as FIG. 2a, for 5 minutes. Three flavorless objects (objects O1, O2 & O3) with the same size, color, shape and material are respectively fixed at three corners of the open box.

On the 27^{th} day, the rat "M" under the test is first placed in the open box shown as FIG. 2a, and the time spent exploring the object O1 (T_{O1}) and the total time spent exploring the objects O1, O2 & O3 (T_{O1+O2+O3}) are recorded, respectively. The percentage of the exploration time spent on the object O1 is calculated as (T_{O1}/T_{O1+O2+O3})*100%. After 5 minutes, a novel object O4 with different size, color, shape and material is used to replace the object O1 (shown in FIG. 2b), and the rat "M" under the test is placed in the open box. The time spent exploring the novel object O4 (T_{O4}) and the total time spent exploring the objects O2 & O3 and the novel object O4 (T_{O2+O3+O4}) are respectively recorded, and the percentage of the exploration time spent on the novel object O4 is calculated as (T_{O4}/T_{O2+O3+O4})*100%.

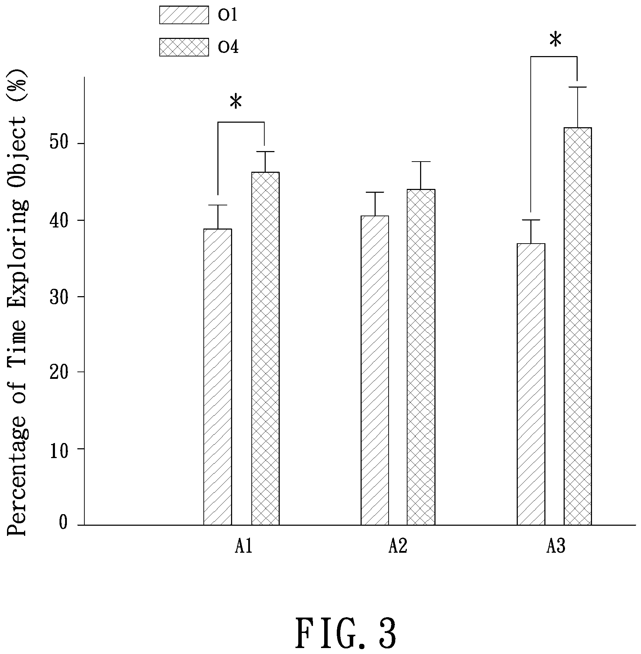

Referring to FIG. 3, the normal rats of group A1 significantly spend more time exploring the novel object O4 than exploring the object O1 (*p*<0.05), indicating that the normal rats can recognize the novel object O4 in the environment. Moreover, according to the result of DLB rats of group A2 (saline), the DLB rats have the cognitive decline on recognition ability and cannot recognize the novel object O4. In addition, the DLB rats of group A3 (receiving ceftriaxone treatment) can recognize the novel object O4 in the environment (*p*<0.05), indicating the administration of ceftriaxone can significantly treat the cognitive decline on recognition ability of the DLB rats.

### Trial (C). Active Avoidance Test

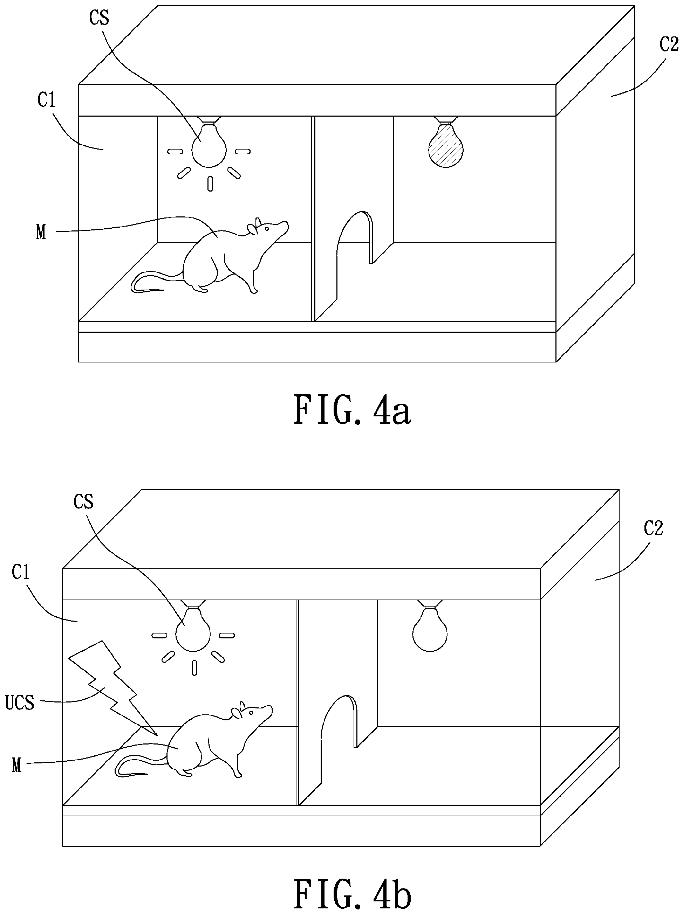

To measure the learning ability of the rats, the active avoidance test is used on the 26^{th} day. The rat "M" under the test is placed in a shuttle box, shown in FIG. 4a, with two compartments intercommunicating with each other.

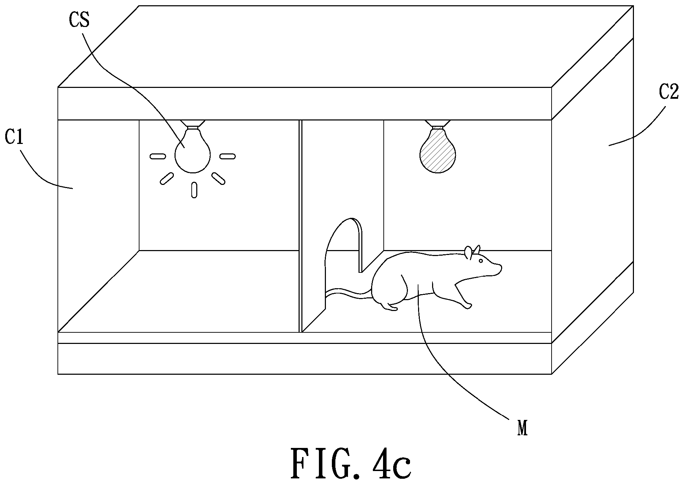

Referring to FIG. 4a, the rat "M" under the test is placed in a compartment C1 of the shuttle box, and is then given a conditioned stimulus (CS) including light and sound for 3 seconds. If the rat "M" under the test does not move to the opposite compartment C2 during the CS presentation as shown in FIG. 4b, the rat "M" under the test is then given an unconditioned stimulus (UCS) being a footshock for 10 seconds. Thus, the rat "M" under the test will learn to move to the compartment C2 during the CS presentation, as shown in FIG. 4c, in order to avoid the subsequent UCS (so-called the active avoidance response). The active avoidance test consists of 25 rounds, and the frequency of the rat "M" under the test successfully avoiding the UCS (% of active avoidance) in the 25 rounds is recorded.

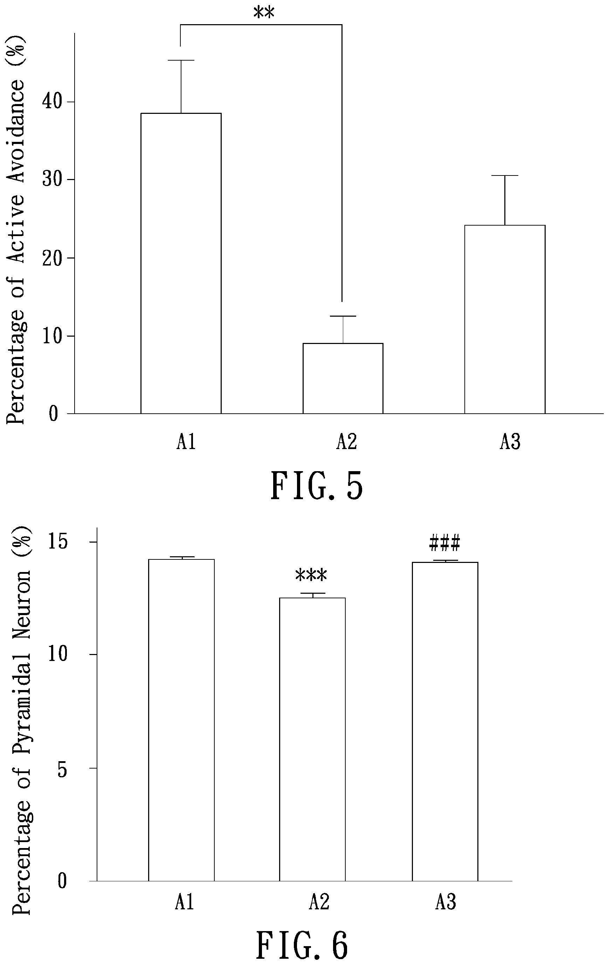

Referring to FIG. 5, compared to the normal rats of group A1, the DLB rats of group A2 (saline) have a lower frequency of avoiding the UCS (*p*<0.01). While, compared to the normal rats of group A1, the DLB rats of group A3 (receiving ceftriaxone treatment) have a similar frequency of avoiding the UCS, suggesting the administration of ceftriaxone can significantly treat the cognitive decline on learning ability of the DLB rats.

### Trial (D). Histopathological Analysis

On the 27^{th} day, after the behavioral tests, BrdU (bromodeoxyuridine) is IP injected into the rats of groups A1-A3 to label the newly generated cells. Moreover, on the 28^{th} day, the rats of groups A1-A3 are sacrificed and the coronal brain sections are collected. The sections with hippocampal CA1 area are used for Nissl staining. The percentage of pyramidal neuron (Nissl-stained cells) in a predetermined area in the hippocampal CA1 area is shown in FIG. 6.

Referring to FIG. 6, compared to the normal rats of group A1, the DLB rats of group A2 (saline) have a significantly decreased percentage of pyramidal neuron in the predetermined area in the hippocampal CA1 area (*p*<0.001). Moreover, compared to the DLB rats of group A2 (saline), the DLB rats of groups A3 (receiving ceftriaxone treatment) show increased percentage of pyramidal neuron in the predetermined area in the hippocampal CA1 area (*p*<0.001).

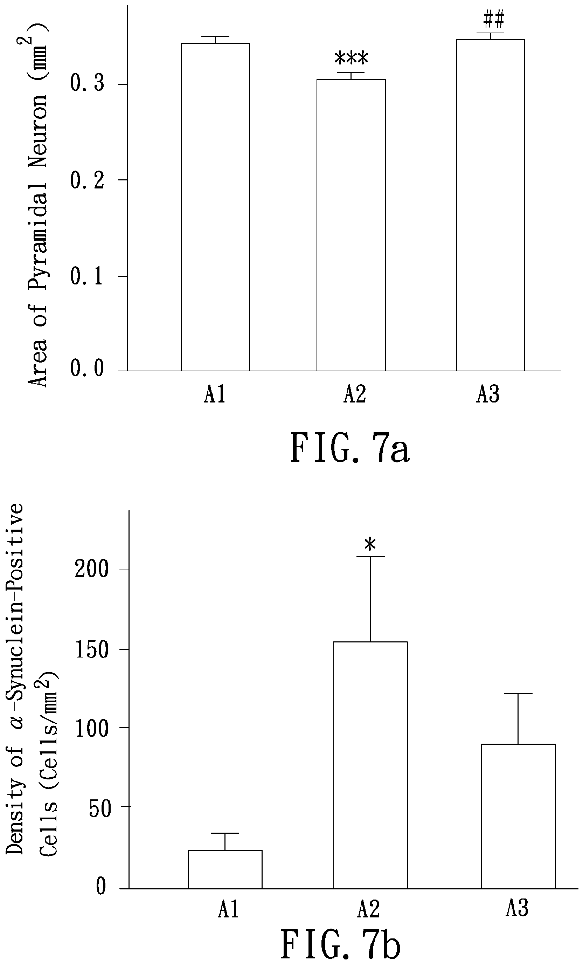

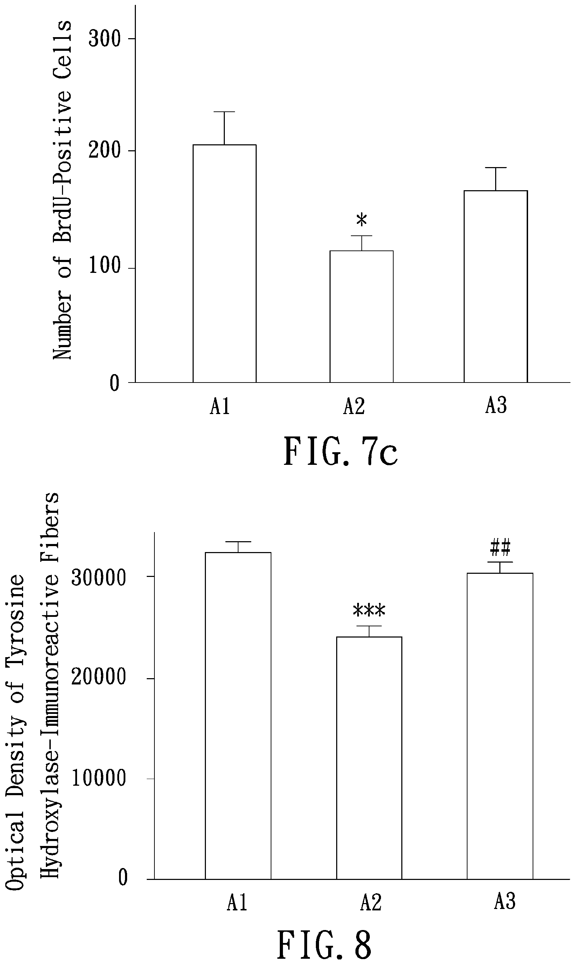

The sections with dentate gyrus (DG) are used for Nissl staining, α-synuclein staining and BrdU staining. The area of pyramidal neuron (Nissl-stained cells), the density of α-synuclein-positive cells and the number of BrdU-positive cells in the predetermined area in DG are shown in FIGS. 7a, 7b and 7c, respectively.

Referring to FIG. 7a, compared to the normal rats of group A1, the DLB rats of group A2 (saline) have a significantly decreased area of pyramidal neuron in the predetermined area in DG (*p*<0.001). Moreover, compared to the DLB rats of group A2 (saline), the DLB rats of group A3 (receiving ceftriaxone treatment) show increased area of pyramidal neuron in the predetermined area in DG (*p*<0.01).

Referring to FIG. 7b, compared to the normal rats of group A1, the DLB rats of group A2 (saline) have a significantly increased density of α-synuclein-positive cells in the predetermined area in DG (*p*<0.05). Moreover, compared to the normal rats of group A1, the DLB rats of group A3 (receiving ceftriaxone treatment) show a similar density of α-synuclein-positive cells in the predetermined area in DG.

Referring to FIG. 7c, compared to the normal rats of group A1, the DLB rats of group A2 (saline) have a significantly decreased number of BrdU-positive cells in the predetermined area in DG (*p*<0.05), indicating the inhibition of neurogenesis in the DLB rats. Moreover, compared to the normal rats of group A1, the DLB rats of group A3 (receiving ceftriaxone treatment) have a similar number of BrdU-positive cells in the predetermined area in DG.

The brain sections with striatum are used for tyrosine hydroxylase staining. The optical density of tyrosine hydroxylase-immunoreactive fibers in the predetermined area in striatum is shown in FIG. 8.

Referring to FIG. 8, compared to the normal rats of group A1, the DLB rats of group A2 (saline) have a significantly decreased optical density of tyrosine hydroxylase-immunoreactive fibers in the predetermined area in striatum (*p*<0.001). Moreover, compared to the DLB rats of group A2 (saline), the DLB rats of group A3 (receiving ceftriaxone treatment) have an increased optical density of tyrosine hydroxylase-immunoreactive fibers in the predetermined area in striatum (*p*<0.01).

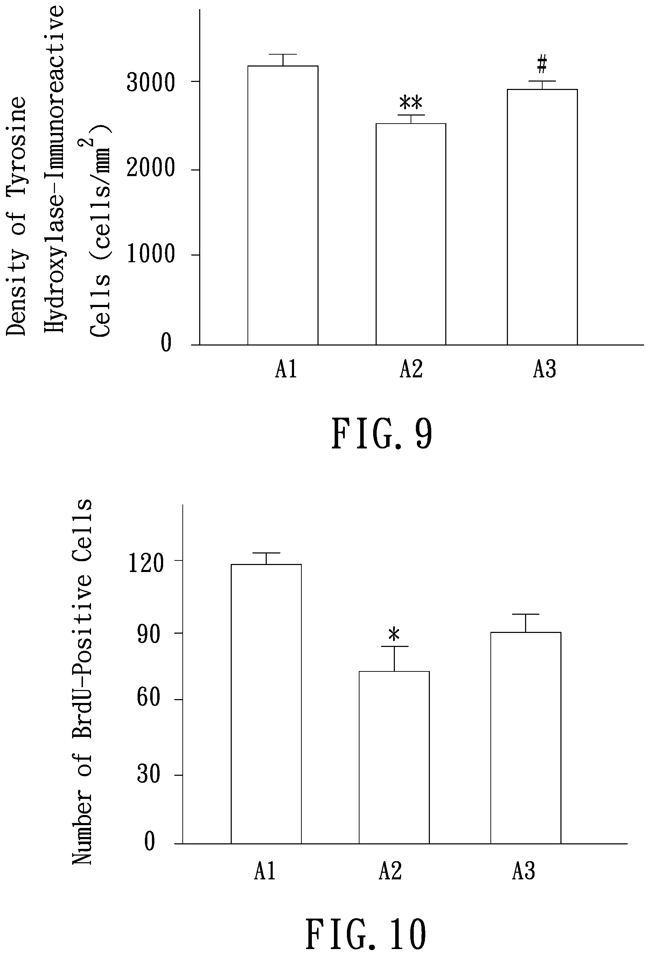

The brain sections with substantia nigra pars compacta (SNc) are used for tyrosine hydroxylase staining. The density of tyrosine hydroxylase-immunoreactive cells (dopaminergic neurons) in the predetermined area in SNc is shown in FIG. 9.

Referring to FIG. 9, compared to the normal rats of group A1, the DLB rats of group A2 (saline) have a significantly decreased density of dopaminergic neurons in the predetermined area in SNc (*p*<0.01). Moreover, compared to the DLB rats of group A2 (saline), the DLB rats of group A3 (receiving ceftriaxone treatment) have an increased density of dopaminergic neurons in the predetermined area in SNc (*p*<0.05).

The brain sections with substantia nigra pars reticulata (SNr) are used for BrdU staining. The number of BrdU-positive cells in the predetermined area in SNr is shown in FIG. 10.

Referring to FIG. 10, compared to the normal rats of group A1, the DLB rats of group A2 (saline) have a significantly decreased number of BrdU-positive cells (*p*<0.05). Moreover, compared to the normal rats of group A1, the DLB rats of group A3 (receiving ceftriaxone treatment) have a similar number of BrdU-positive cells.

Besides, the aforesaid dosage can be converted into a dosage suitable for a human subject according to the dose translation formula based on body surface area (Shannon R.S. et al. (2007), FASEB J., 22: 659-661), suggesting that 0.81-35 mg/kg/day of ceftriaxone can treat the DLB syndromes such as cognitive decline and neurological deficit in the human subject thereof.

In conclusion, by the administration of ceftriaxone, the neurogenesis is improved in the DG and SNr, and the DLB syndromes such as cognitive decline (on recognition ability and learning ability) and neurological deficit are therefore improved.

## Claims

1. Ceftriaxone for use in the treatment of dementia with Lewy bodies (DLB), wherein ceftriaxone is to be administrated to a subject in need thereof to treat syndromes such as cognitive decline and neurological deficit in the subject in need thereof.

2. Ceftriaxone for use in the treatment of DLB as claimed in claim 1, wherein ceftriaxone is to be administrated to the subject in a dosage of 1.5-35 mg/kg/day.

3. Ceftriaxone for use in the treatment of DLB as claimed in claim 2, wherein ceftriaxone is to be administrated to the subject in a dosage of 1.62-32.4 mg/kg/day.

4. Ceftriaxone for use in the treatment of DLB as claimed in claim 3, wherein ceftriaxone is to be administrated to the subject in a dosage of 1.62-16.2 mg/kg/day.

5. Ceftriaxone for use in the treatment of DLB as claimed in claim 4, wherein ceftriaxone is to be administrated to the subject in a dosage of 1.62-8.1 mg/kg/day.

6. Ceftriaxone for use in the treatment of DLB as claimed in claim 1, wherein ceftriaxone is to be parenterally or orally administrated to the subject.

7. Ceftriaxone for use in the treatment of DLB as claimed in claim 6, wherein ceftriaxone is to be administrated to the subject by intravenous injection, intramuscular injection, intraperitoneal injection, transdermal administration, sublingual administration or hebulization administration.
